# EUROPÄISCHE PATENTANMELDUNG

(11) **EP 1 964 576 A1**
(43) Veröffentlichungstag der Anmeldung: **03.09.2008**
(21) Anmeldenummer: 07003983.9
(22) Anmeldetag: 27.02.2007
(51) Int. Cl.: A61K 47/10, A61K 47/32, A61K 9/00, A61K 36/28, A61K 36/537

(54) **Wässrige, desinfizierende Gelzubereitung**

(71) Anmelder: Certmedica International GmbH, 63801 Kleinostheim (DE)
(72) Erfinder: Raisch, Kurt, 61381 Friendrichsdorf im Taunus (DE)
(74) Vertreter: Pfortner, Peter

(57) **Zusammenfassung**

Die vorliegende Erfindung betrifft eine wässrige, desinfizierende Gelzubereitung, die 40 bis 80 Gew.-% mindestens eines alkoholischen Wirkstoffes; 0,1 bis 15 Gew.-% mindestens eines Verdickungsmittcls; und 0,1 bis 10 Gew.-% mindestens einer weiteren Wirkstoflkomponente unifasst, ausgewählt aus der Gruppe, die besteht aus beruhigender, heilungsiördernder, lindernder, pflegender und/oder entzündungshemmender Komponente, wobei der Rest, um 100 Gew.-% zu ergeben, gereinigtes oder destilliertes Wasser ist. Darüber hinaus betrifft die vorliegende Erfindung ein Verfahren zu ihrer Herstellung sowie die Verwendung der erfindungsgemäßen Gelzubereitung zur Desinfektion von Haut oder Händen.

## Beschreibung

Die vorliegende Erfindung betrifft eine wässrige, desinfizierende Gelzubereitung, ein Verfahren zu ihrer Herstellung sowie die Verwendung der Gelzubereitung zur Desinfektion von Haut oder Händen. Insbesondere betrifft die vorliegende Erfindung eine wässrige, desinfizierende Gelzubereitung mit heilenden, lindernden, pflegenden und/oder entzündungshemmenden Wirkstoffen.

Desinfektionsmittel dienen der Bekämpfung pathogener Mikroorganismen wie z.B. Bakterien, Viren, Sporen, Pilze etc., und ihr Einsatz ist in vielen Bereichen unverzichtbar.

Das Anwendungsgebiet der vorgenannten Desinfektionsmittel kann medizinisch indiziert sein und der Prävention von Infektionen in Krankenhäusern, Arztpraxen, Pflegeeinrichtungen, z.B. Altenheimen, Sanatorien etc., Sportstätten sowie in anderen Bereichen einschließlich privater Bereiche dienen, in denen Infektionen übertragen werden können. Darüber hinaus ist neben dem Einsatz von Desinfektionsmitteln in der Lebensmittel- und Pharmaindustrie auch deren Verwendung in Dienstleistungseinrichtungen wie z.B. Wäschereien oder Küchen als Beispiel zu nennen.

Im Markt erhältliche Hand- und Hautdesinfektionsmittel bestehen üblicherweise aus Alkohol oder Mischungen aus Alkoholen sowie optional weiteren Wirkstoffen, die nach dem Verdunsten der Alkohol-Komponente(n) auf der Haut verbleiben.

Nachteil derartiger alkoholischer Desinfektionslösungen bei der Applikation für die Händedesinfektion ist ihre schwierige Dosierung, weil die benötigte Desinfektionsmittelmenge vielfach nicht gleichmäßig über die Haut oder Hände verteilt werden kann, da die Alkohollösung sehr leicht von der Haut oder den Händen tropft. Um ein Tropfen zu reduzieren und somit die Kontaktzeit des Desinfektionsmittels mit den zu desinfizierenden Stellen zu erhöhen, wurden alkoholischen Desinfektionslösungen Verdickungsmittel zugesetzt. So wurde die Viskosität erhöht. Beispielsweise ist in der Druckschrift EP 0 604 848 eine schnelltrocknende Desinfektionsmittel-Zusanunensetzung beschrieben. Die weist typischerweise einen Alkoholgehalt von 40 bis 90 % auf und schließt darüber hinaus als Verdickungsmittel eine Kombination aus Carboxyvinyl-Polymeren und Hydroxypropylmethylcellulose ein.

Antimikrobielle alkoholische Gelzusammensetzungen zur Händedesinfektion, die Feuchthaltcmittel enthalten, werden auch in der Druckschrift US-A 4 956 170 beschrieben. Als Verdickungsmittel werden in diesen Zusammensetzungen teilneutralisierte bzw. neutralisierte Acrylsäurepolymere eingesetzt.

Die Druckschriften EP 0 320 254 und US-A 5 098 717 offenbaren wässrige, bakterizide oder bakeriostatische Gelzubereitungen, die etwa 60 bis etwa 90 Gew.-% Alkohol, etwa 0,5 bis etwa 30 Gew.-% Wasser, etwa 0,5 bis etwa 5 Gew.-% mindestens eines Gelierungsmittels und darüber hinaus eine wirksame Menge eines topisch aktiven Wirkstoffs umfassen.

Den desinfizierenden Gelzubereitungen des Standes der Technik gemeinsam ist ein hoher Alkoholgehalt. Insbesondere dann, wenn Alkohol, der aufgrund seiner hohen Flüchtigkeit nach dem Aufbringen sehr schnell verdampft, die einzige Wirkkomponente ist, ist ein hoher Alkoholgehalt erforderlich, um einer wirksame Haut- bzw. Händedesinfektion sicherzustellen.

Ein hoher Gehalt an Alkohol hat jedoch ein Austrocknen der Haut zur Folge. Dies ist insbesondere in solchen Fällen nachteilig, in denen eine Händedesinfektion mehrere Male am Tag durchgeführt werden muß, beispielsweise in Krankenhäusern, auf Pflegestationen oder in der Lebensmittelindustrie. Durch die häufige Verwendung herkömmlicher Desinfektionsmittel mit einem hohen Alkoholgehalt kann es zu gesundheitlichen Beinträchtigungen kommen, die auf das Austrocknen der Haut und/oder permanente Hautirritationen zurückzuführen ist. Zur Lösung dieses Problems schlägt die Druckschrift EP 0 604 848 die Verwendung eines Feuchthaltemittels vor. Jedoch hat sich in der Praxis herausgestellt, dass der Zusatz eines Feuchthaltemittels allein die Probleme, die mit dem Austrocknen der Haut und/oder permanenten Hautirritationen einhergehen, nicht zufriedenstellend gelöst werden können.

Es war deshalb eine Aufgabe der vorliegenden Erfindung, eine desinfizierende Gelzubereitung mit hohem Alkoholgehalt zur Haut- oder Händedesinfektion bereitzustellen, die eine wirksame Desinfektion sicherstellt und gleichzeitig die Nachteile der bisher bekannten Desinfektionsmittel mit hohem Alkoholgehalt, nämlich ein Austrocknen der Haut sowie permanente Hautirritationen, überwindet.

Diese und andere Aufgaben der vorliegenden Erfindung werden gelöst durch eine wässrige, desinfizierende Gelzubereitung, die 40 bis 80 Gew.-% mindestens eines alkoholischen Wirkstoffes 0,1 bis 15 Gew.-% mindestens eines Verdickungsmittels und 0,1 bis 10 Gew.-% mindestens einer weiteren Wirkstoffkomponente umfaßt, ausgewählt aus der Gruppe, die besteht aus beruhigender, heilungsfördernder, lindernder, pflegender und entzündungshemmender Komponente, wobei der Rest, um 100 Gew.-% zu ergeben, Wasser ist.

Die wässrige, desinfizierende Gelzubereitung schließt als eine der Komponenten mindestens einen alkoholischen Wirkstoff, vorzugsweise einen oder zwei alkoholische(n) Wirkstoff(e), noch weiter bevorzugt einen alkholischen Wirkstoff, in einer Menge im Bereich von 40 bis 80 Gew.-%, bevorzugt im Bereich von 50 bis 75 Gew.-% ein, bezogen auf die Gesamtmenge der Gelzubereitung. Am meisten bevorzugt liegt die Konzentration der mindestens einen alkoholischen Wirkstoffkomponente in einer Menge im Bereich von 60 bis. 70 Gew.-% enthalten. Die Menge des alkoholischen Wirkstoffs bzw. der alkoholischen Wirkstoffe, der/die in der erfindungsgemäßen desinfizierenden Gelzubereitung enthalten ist/sind, wird so ausgewählt, dass damit einerseits eine schnelle und wirksame Abtötung der Mikroorganismen sichergestellt wird und andererseits die Stabilität der Gelzubereitung nicht beeinträchtigt wird.

Die Alkoholkomponente, insbesondere in den o.g. Mengenbereichen, gewährleistet ein schnelles und wirksames Abtöten von Mikroorganismen. Alkohol verringert sehr schnell die Anzahl der Keime (mikrobielle "counts"), unmittelbar nachdem er auf die zu desinfizierenden Stellen aufgetragen wurde. Als alkoholische Wirkstoffkomponente(n), die zur Verwendung auf der Haut oder den Händen geeignet ist/sind, kommt/kommen ein oder mehrere Alkohol(e) aus der Gruppe Ethanol, 1-Propanol, 2-Propanol (Isopropylalkohol), 1-Butanol, Isobutylalkohol, sec. Butylalkohol, tert. Butylalkohol, 1-Pentanol, 2-Pentanol, 3-Pentanol und Neopentylalkohol zum Einsatz. Die vorgenannten Alkohole werden allein oder in Mischungen aus zwei oder mehreren verwendet werden. Ethanol und/oder Isopropanol sind besonders bevorzugt. Am meisten bevorzugt ist technischer Alkohol, d.h. Alkohol, der mindestens 92 Gew.-% Ethanol enthält, oder absoluter Alkohol mit mindestens 99,7 Vol.% Ethanol. Besonders bevorzugt ist Neutralalkohol (Ethanol: 96 Vol-%), der mit Phthalsäurediethylester vergällt ist.

Die erfindungsgemäße wässrige, desinfizierende Gelzubereitung weist eine Viskosität von 1,0 bis 8,0 Pa.s auf Bevorzugt ist eine Viskosität von 2 bis 6,5 Pa.s. Die Einstellung der Viskosität erfolgt über die Menge mindestens eines Verdickungsmittel, das als weitere Komponente in der erfindungsgemäßen Gelzubereitung enthalten ist, und/oder durch Einstellen eines bestimmten pH-Wertes der Gelzubereitung. Die Menge des/der verwendeten Verdickungsmittel(s) liegt im Bereich von von 0,1 bis 15 Gew.-%, bevorzugt im Bereich von 0,5 bis 5 Gew.-% und am meisten bevorzugt im Bereich von 0,6 bis 1,5 Gew.-%, bezogen auf die Gesamtmenge der Gelzubereitung.

Die resultierende Gelzubereitung verringert ein Herablaufen der Desinfektionsmittel-Zubereitung durch Ausbildung eines Films auf der Haut. Dadurch verbleibt sie an der Stelle, an der eine Desinfektionswirkung erwünscht ist, wodurch gleichzeitig die Kontaktzeit des Desinfektionsmittels mit der Haut oder den Händen erhöht werden kann.

Bevorzugte Verdickungsmittel, die in der erfindungsgemäßen Gelzubereitung verwendet werden, sind wasserlöslich und mit dem Alkohol kompatibel, der in der Gelzubereitung verwendet wird, und können jedes Mittel sein, das eine stabile Gelmatrix in Gegenwart von Alkohol und Wasser hervorruft.

Als erfindungsgemäß einzusetzende Verdickungsmittel sind im Handel erhältliche vollsynthetische Verdickungsmittel auf der Basis von Acrylsäuze-Copolymeren, Methacrylsäure-Copolymeren, Vinylpolymeren, Polycarbonsäuren, Polyiminen, Polyamiden und Polyethern zu nennen. Daneben können auch natürliche Verdickungsmittel wie beispielsweise Carboxymethylcellulosen, Celluloseether, Xanthane, Guar-Mehl, Johannesbrotbaumkernmehl eingesetzt werden, die gegebenenfalls durch geeignete chemische Reaktionen modifiziert werden, gegebenenfalls sogar im Hinblick auf ihre konkrete Verwendungsweise im Zusammenhang mit der vorliegenden Erfindung. Die vorgenannten Komponenten können entweder allein oder in Kombinationen von zwei oder mehreren von ihnen verwendet werden.

Veranschaulichende Beispiele von im Handel erhältlichen Verdickungsmitteln sind Produkte, die unter der Bezeichnung Carbopol (z.B. Carbopol ULTREZ 10, Carbopol EZ 2, Carbopol ETD 2001, Carbopol ETD 2020, Carbopol ETD 2050, Carbopol 934, Carbopol 2984, Carbopol 940 und Carbopol 1342), vertrieben von der Firma B.F. Goodrich, unter der Bezeichnung Natrosol, vertrieben von der Firma Broste, Dänemark, unter der Bezeichnung Satiaxane, vertrieben von der Firma Superfos, Dänemark, unter der Bezeichnung Celacol, vertrieben von der Firma Superfos, Dänemark, unter der Bezeichnung Courlose, vertrieben von der Firma Superfos, Dänemark, und unter der Bezeichnung Cekol, vertrieben von der Firma Superfos, Dänemark, erhältlich sind.

Vorzugsweise ist das mindestens eine Verdickungsmittel ausgewählt aus der Gruppe, die besteht aus Carbopol Ultrez 10 (ein vernetztes Acrylsäure-Polymer), CarboxyvinylPolymer oder Hydroxypropylmethylcellulose und/oder Kombinationen davon.

Da der hohe Alkoholanteil in der desinfizierenden Gelzubereitung, insbesondere bei häufiger Applikation, ein Austrocknen der Haut oder Hautirritationen hervorrufen kann, die zu gesundheitlichen Beeinträchtigungen, wie beispielsweise Juckreiz, Hautrötungen und Ekzemen, führen können, ist der Einsatz mindestens einer weiteren Wirkstoffkomponente zur Vorbeugung oder Therapie solcher Erkrankungen erfindungsgemäß vorgesehen. Erfindungsgemäß wird/werden daher der Gelzubereitung ein oder mehrere weitere(r) Wirkstoff(e) zusetzt, der/die nach dem Verdunsten der Alkoholkomponente auf der Haut verbleibt/verbleiben. Derartige Komponenten haben eine beruhigende, heilungsfördernde, lindernde, pflegende und/oder entzündungshemmende Wirkung.

Letztgenannte Wirkstoffe sind in den Endprodukten allein oder in Kombination üblicherweise in einer Menge im Bereich von 0,1 bis 10,0 Gew.-% enthalten, vorzugsweise in einer Menge im Bereich von 0,1 bis 5,0 Gew.-% und am meisten bevorzugt in einer Menge im Bereich von 0,1 bis 2,5 Gew.-%. Der Begriff "in Kombination" bedeutet in diesem Zusammenhang, dass entweder mehrere Wirkstoffe aus der vorstehend genannten Gruppe verwendet werden können, die derselben Gruppe von Komponenten angehören (z. B. mehrere heilungsfördernde Wirkstoffe oder mehrere pflegende Wirkstoffe), oder daß mehrere Wirkstoffe aus der vorstehend genannten Gruppe verwendet werden können, die verschiedenen Gruppen von Komponenten angehören (z. B. eine oder mehrere heilungsfördernde Komponente(n) zusammen mit einem oder mehreren lindernden Komponente(n)).

Als lindernde oder heilungsfördernde Komponenten, die vorzugsweise allein oder in Kombination in der erfindungsgemäßen desinfizierenden Gelzubereitung enthalten sind, sind Kamillenextrakt (z. B. das Produkt Dragosantol 100, erhältlich von der Firma Symrise), Amikatinktur, Extrakte aus Teebaum, Lavendel, Ringelblume, Rosmarin, Nelken, Hamamelis, Wacholder, Klette, Hopfen, Schafgarbe, Spitzwegerich, Zeder, Pinie und/oder Eukalyptus zu nennen.

Bisabolol (1-Methyl-4-(1,5-dimethyl-1-hydroxyhex-4(5)-enyl-)cyclohexen-1 ist ein etherisches Öl, das in der Kamille vorkommt und das in niederen Alkoholen wie beispielsweise Ethanol, Isopropanol gut löslich ist. Das hauptsächlich in der Kosmetik verwendete racemische Gemisch aus 1- und d-α-Bisabolol wird aus Zwischenprodukten der Vitaminsynthese technisch hergestellt. Bisabolol zeigt entzündungshemmende (antiphlogistische antiproliferative) Wirkungen. Daneben spielen auch die antibakteriellen Eigenschaften der Substanz eine Rolle für den Einsatz in kosmetischen Mitteln.

Vorteilhaft ist weiterhin die Zugabe von Arnika in Form einer Tinktur, die aus Arnikablüten gewonnen wird. Die Tinktur kann beispielsweise aus 1 Teil Droge und 10 Teilen Ethanol durch ein geeignetes Verfahren hergestellt werden. Das Haupt-Einsatzgebiet der Arnika ist die äußerliche Anwendung vor allem gegen stumpfe Verletzungen wie Prellungen und Verstauchungen. Daneben hat Arnika auch bei Ekzemen eine heilende Wirkung.

In einer weiteren Ausführungsform kann die mindestens eine weitere Wirkstoffkomponente der desinfizierenden Gelzubereitung eine entzündungshemmende Komponente sein, die ausgewählt ist aus der Gruppe, die besteht aus Salbei-Tinktur, Extrakte aus Teebaum, Lavendel, Ringelblume, Rosmarin, Nelken, Hamamelis, Wacholder, Klette, Hopfen, Schafgarbe, Spitzwegerich, Zeder, Pinie und Eukalyptus.

Salbei ist eine Gattung der Lippenblütengewächse. Die Hauptwirkstoffe sind die etherischen Öle Thujon und Cineol, Gerbstoffe sowie Bitterstoffe. Durch den hohen Anteil etherischer Öle gibt es unterschiedliche Verwendungen. Vom Salbei ist heute allgemein bekannt, dass die Inhaltsstoffe im Allgemeinen sekretionsfördernd, antibakteriell und entzündungshemmend wirken.

In der erfindungsgemäßen desinfizierenden Gelzubereitung wird Salbei in Form einer Salbei-Tinktur eingesetzt, und zwar reichlich in Wasser verdünnt, beispielsweise in Form einer 70 %igen Salbei-Tinktur mit einer Verdünnung von 1 : 10. Zur Herstellung der Tinktur werden Salbeiblätter mit Ethanol extrahiert. Der so erhaltene Extrakt wird mit Wasser verdünnt.

Aufgrund ihres hohen Alkoholgehaltes einerseits und der Gegenwart von Wasser andererseits fungiert die erfindungsgemäße Gclzubereitung auch als Penetrationsverstärker für die aktiven pharmazeutischen Bestandteile, wie beispielsweise die o.g. heilungsfördernden, lindernden, pflcgenden und/oder entzündungshemmenden Wirkstoffkomponenten, indem sie das Stratum Corneum verstärken und dessen Permeabilitätsvermögen erhöhen.

In einer besonderen Ausführungsform der vorliegenden Erfindung umfasst die wässrige, desinfizierende Gelzubereitung darüber hinaus mindestens einen Feuchthaltewirkstoff, um sowohl ein Austrocknen der Gelzubereitung als auch ein Austrocknen der Haut oder der Hände zu verhindern. Geeignete Beispiele derartiger Feuchthaltewirkstoffe sind Glycerin, Propylenglykol, Sorbit, 1,3-Butylenglykol, Polyethylenglykol, Pentandiol, Hyaluronsäure, Harnstoff und Natriumpynolidoncarboxylat, die allein oder in Kombination aus zwei oder mehreren davon verwendet werden. Der Gehalt des Feuchthaltewirkstoffes hängt natürlich sehr stark vom Erfordernis ab, der Haut oder den Händen Feuchtigkeit zuzuführen, was wiederum von der Häufigkeit der Anwendung der Desinfektionsmittel-Zubereitung abhängt, und liegt typischerweise bei einer Menge im Bereich von 1,0 bis 8,0 Gew.-%, bezogen auf das Gesamtgewicht der erfindungsgemäßen Gelzubereitung. Bevorzugt ist ein Gehalt an Feuchthaltewirkstoff im Bereich von 2,0 bis 6,0 Gew.-%, und am meisten bevorzugt im Bereich von 3,0 bis 4,0 Gew.-%. Besonders bevorzugt unter den o.g. Verbindungen sind Polyole und am meisten bevorzugt ist 1,2-Propylenglykol.

Da die erfindungsgemäße wässrige, desinfizierende Gelzubereitung auf die Haut oder die Hände aufgetragen wird, sollte die Zubereitung vorzugsweise einen pH-Wert im Bereich von 4 bis 9, wie beispielsweise im Bereich von 4,5 bis 8, vorzugsweise im Bereich von 5 bis 7,5, aufweisen.

Die Einstellung des pH-Wertes und das Gelieren der erfindungsgemäßen desinfizierenden Gelzubereitung erfolgt über mindestens ein Neutralisationsmittel. Als Neutralisationsmittel werden übliche basische organische Verbindungen verwendet. Vorzugsweise werden Isopropylamin, Aminomethylpropanol, das beispielsweise unter der Bezeichnung AMP Ultra PC 2000 im Handel erhältlich ist, Aminomethylpropandiol, Triethanolamin, Diisopropanolamin und/oder Tetrahydroxypropylethylendiamin und Mischungen aus zwei oder mehreren davon eingesetzt, bis ein pH-Wert im oben angegebenen Bereich in der Endzubereitung erreicht ist. Der Gehalt des mindestens einen Neutralisationsmittels liegt typischerweise in einem Bereich von 0,1 bis 2 Gew.-%, vorzugsweise in einem Bereich von 0,5 bis 1,5 Gew.-% und am meisten bevorzugt in einem Bereich von 0,75 bis 1,25 Gew.-% bezogen auf das Gesamtgewicht der erfindungsgemäßen Gelzubereitung.

Der Zusatz von mindestens einer weiteren desinfizierenden Komponente dient dazu, die desinfizierenden Eigenschaften der Alkoholkomponente zur verstärken. Allerdings ist der Zusatz solcher desinfizierender Komponenten nicht zwingend erforderlich, da die erfindungsgemäßen wässrigen, desinfizierenden Gelzubereitungen einen so hohen Alkoholgehalt aufweisen, dass die Alkoholkomponente des resultierenden Desinfektionsgels allein als desinfizierender Wirkstoff ausreicht. Derartige zusätzliche desinfizierende Komponenten sind beispielsweise Invertseifen, Biguanidverbindungen, Phenolverbindungen, Iodverbindungen, Pigmentverbindungen und beliebige Mischungen aus diesen Komponenten.

Weitere Komponenten, die der wässrigen, desinfizierenden Gelzubereitung optional zugesetzt werden können, sind beispielsweise Lösungsvcrmittler, Rückfetter, etherische Öle, Duftstoffe, Farbstoffe, Konservierungsmittel und/oder Filmbildner. Der Gehalt dieser Komponenten kann im einzelnen im Bereich von 0 bis 5 Gew.-% oder bei 0,1 bis 2 Gew.-% liegen und liegt typischerweise insgesamt in einem Bereich von 0,2 bis 1,0 Gew.-%, jeweils bezogen auf das Gesamtgewicht der erfindungsgemäßen Gelzubereitung.

Zur Solubilisierung der etherischen Öle und Parfümöle werden der erfindungsgemäßen wässrigen, desinfizierenden Gelzubereitung Lösungsvermittler zugesetzt. Beispielhaft ist der nichtionogene Lösungsvermittler 2014160, erhältlich von der Firma Cosnaderm, zu nennen, bei dem es sich um ein Gemisch aus Fettalkoholpolyglykolether und hydriertem Rhizinusölethoxylat handelt. Der Lösungsvermittler wird typischerweise in einer Menge im Bereich von 0,05 bis 2,0 Gew.-% verwendet.

Vorzugsweise schließt die erfindungsgemäße wässrige, desinfizierende Gelzubereitung darüber hinaus einen Rückfetter ein, der als Pflegewirkstoff fungiert. Insbesondere wird ein Rückfetter in die erfindungsgemäße Gelzubereitung eingearbeitet, um ein Sprödewerden der Haut aufgrund der entfettenden Eigenschaft der Alkoholkomponente zu verringern oder zu verhindern. Der Rückfetter ist vorzugsweise gewählt aus der Gruppe, die besteht aus Fettsäurepolydiethanolamiden, Betainen, Lanolin, Paraffinölen, Kokosfettsäurediethanolamiden und Kokosfettsäuremonoethanolamiden. Als besonders geeignet hat sich Polyethylenglykolmonotridecylether, der unter der Bezeichnung NEO-PCL Wasserlöslich N im Handel erhältlich ist, herausgestellt.

Schließlich enthalten die wässrigen, desinfizierenden Gelzubereitungen gemäß der vorliegenden Erfindung Wasser im Bereich von 15,0 bis 45,0 Gew.-%, bezogen auf das Gesamtgewicht der Gesamtzubereitung. Das Wasser wird vorzugsweise in gereinigter, destillierter oder entionisierter Form eingesetzt.

Zur Herstellung der erfindungsgemäßen wässrigen, desinfizierenden Gelzubereitung wird zunächst die Alkoholkomponente mit der erforderlichen Menge Wasser gemischt. Im Anschluss daran werden die meist in alkoholisch wässrigen Medien gut löslichen Wirkstoffkomponenten zugegeben. In diese Mischung wird gegebenenfalls der Lösungsvermittler eingebracht und danach die weiteren Komponenten (Rückfetter, Aromen, Duftstoffe, Farbstoffe) zugegeben. Nach Zugabe des Verdickungsmittel wird die Mischung homogenisiert und, falls erforderlich, der pH-Wert eingestellt.

Die Vorteile der erfindungsgemäßen wässrigen, desinfizierenden Gelzubereitungen sind eine stabile, desinfizierende Haut- oder Händedesinfektion mit hohem Alkoholgehalt, die ohne weitere zusätzliche antimikrobielle Zusatzstoffe sowohl die Normen DIN/EN 1499-Desinfizierende Händewaschung als auch DIN/EN 1500-Hygienische Händedesinfektion erfüllen und gleichzeitig eine beruhigende, heilende, lindernde, pflegende und/oder entzündungshemmende Wirkung aufweisen.

Bei dem Verfahren zur Desinfektion von Haut oder Händen wird die wässrige, desinfizierende Gelzubereitung auf die zu desinfizierende Stelle aufgebracht, und man lässt sie je nach Anforderung der Desinfektion auf die Haut oder die Hände eine vorbestimmte Zeit einwirken.

Die Erfindung wird durch die nachfolgenden Beispiele näher erläutert, die bevorzugte Ausführungsformen betreffen und nicht der Beschränkung der Erfindung, sondern einzig ihrer beispielhaften Erläuterung dienen. Alle Mengenangaben sind Angaben in Gew.-% und beziehen sich auf das Gesamtgewicht einer Zubereitung oder Rezeptur, das mit 100 Gew.-% angenommen wird.

### Beispiele

### Beispiel 1

| **Bestandteil** | **Gew.-%** |
|---|---|
| Alkohol | 70 |
| Arnikaextrakt | 0,2 |
| Kamillenextakt | 0,3 |
| Glycerin | 4 |
| Lösungsvermittler (PEG-40 Hydrogenaled Castor Oil) | 0,1 |
| Aroma | 0,05 |
| Verdickungsmittel (Carbopol Ultrez 10) | 1,2 |
| Wasser | ad 100 |

### Beispiel 2

| **Bestandteil** | **Gew.-%** |
|---|---|
| Alkohol | 70 |
| Arnikaextrakt | 1,0 |
| Kamillenextakt | 0,75 |
| Salbeiextrakt | 0,3 |
| Propyleneglycol Lösungsvermittler | 4 |
| (PEG-40 Hydrogenated Castor Oil) | 0,3 |
| Rückfetter (Polyethylenglykolmonotridecylether) | 2,0 |
| Aroma | 0,05 |
| Neutralisationsmittel (Triethanolamin) | 0,3 |
| Verdickungsmittel (Carbopol Ultrez 10) | 1,2 |
| Wasser | ad 100 |

Die Wirksamkeit der erfindungsgemäßen Zubereitung gegen die üblichen pathogenen Mikroorganismen wie z.B. Bakterien, Viren, Sporen, Pilze etc. wurde mit üblichen Verfahren geprüft und zeigte eine hervorragende Wirksamkeit.

Die Hautverträglichkeit der erfindungsgemäßen Zubereitung wurde in einem vergleichenden repetitiven Epikutantest auf intakter und vorgeschädigter Haut untersucht. Verglichen wurde ein Produkt aus dem Stand der Technik ohne heilende und lindernde Komponenten mit dem erfindungsgemäßen Produkt. Die Kombination von lindernden und heilenden Bestandteilen mit rückfettenden Komponenten zeigte dabei eine hervorragende Hautverträglichkeit verglichen mit einem Produkt aus dem Stand der Technik.

Verträglichkeits- und Wirksamkeitstest belegen damit die Erfüllung der erfindungsgemäßen Aufgabe.

## Patentansprüche

1. Wässrige, desinfizierende Gelzubereitung, umfassend
- 40 bis 80 Gew.-% mindestens eines alkoholischen Wirkstoffes;
- 0,1 bis 15 Gew.-% mindestens eines Verdickungsmittels;
- 0,1 bis 10 Gew.-% mindestens einer weiteren Wirkstoffkomponente, ausgewählt aus der Gruppe, die besteht aus beruhigender, heilungsfördernder, entzündungshemmender Komponente;
wobei der Rest, um 100 Gew.-% zu ergeben, Wasser ist.

2. Wässrige, desinifzierende Gelzubereitung nach Anspruch 1, worin der mindestens eine alkoholische Wirkstoff in einer Menge im Bereich von 50 bis 75 Gew.-% enthalten ist.

3. Wässrige, desinfizierende Gelzubereitung nach Anspruch 1 oder Anspruch 2, worin der mindestens eine alkoholische Wirkstoff ausgewählt ist aus der Gruppe, die besteht aus Ethanol, 1-Propanol, 2-Propanol (Isopropylalkohol), 1-Butanol, Isobutylalkohol, sec. Butanol, tert. Butylalkohol, 1-Pentanol, 2-Pentanol, 3-Pentanol und Neopentylalkohol und Mischungen aus zwei oder mehreren davon, vorzugsweise worin der mindestens eine alkoholische Wirkstoff Ethanol und/oder Isopropanol ist.

4. Wässrige, desinfizierende Gelzubereitung nach einem der vorangehenden Ansprüche 1 bis 3 mit einer Viskosität von 1,0 bis 8,0 Pa.s.

5. Wässrige, desinfizierende Gelzubereitung nach einem der vorangehenden Ansprüche 1 bis 4, worin das mindestens eine Verdickungsmittel in einer Menge im Bereich von 0,5 bis 5,0 Gew.-% enthalten ist.

6. Wässrige, desinfizierende Getzubereitung nach einem der vorangehenden Ansprüche 1 bis 5, worin das mindestens eine Verdickungsmittel ausgewählt ist aus der Gruppe, die besteht aus Acrylsäure-Copolymeren, Methacrylsäurecopolymeren, Vinylpolymeren, Polycarbonsäuren, Polyiminen, Polyamiden, Polyethern, Carboxymethylcellulosen, Celluloseethern, Xanthanen, Guar-Mehl, Johannesbrotbaumkemmehl und Kombinationen davon, vorzugsweise worin das mindestens eine Verdickungsmittel ausgewählt ist aus der Gruppe, die besteht aus Carbopol Ultrez 10 (ein vernetztes Acrylsäure-Polymer), Carboxyvinylpolymer oder Hydroxypropylmethylcellulose und Kombinationen davon.

7. Wässrige, desinfizierende Gelzubereitung nach einem der vorangehenden Ansprüche 1 bis 6, worin die mindestens eine weitere Wirkstoffkomponente in einer Menge im Bereich von 0,1 bis 5 Gew.-% enthalten ist.

8. Wässrige, desinfizierende Gelzubereitung nach einem der vorangehenden Ansprüche 1 bis 7, worin die mindestens eine heilungsfördernde Komponente ausgewählt ist aus der Gruppe, die besteht aus Kamillenextrakt, Arnikatinktur, Extrakte aus Teebaum, Lavendel, Ringelblume, Rosmarin, Nelken, Hamamelis, Wacholder, Klette, Hopfen, Schafgarbe, Spitzwegerich, Zeder, Pinie und Eukalyptus.

9. Wässrige, desinfizierende Gelzubereitung nach einem der vorangehenden Ansprüche 1 bis 8, worin die mindestens eine entzündungshemmende Komponente ausgewählt ist aus der Gruppe, die besteht aus Salbei-Tinktur, Extrakte aus Teebaum, Lavendel, Ringelblume, Rosmarin, Nelken, Hamamelis, Wacholder, Klette, Hopfen, Schafgarbe, Spitzwegerich, Zeder, Pinie und Eukalyptus.

10. Wässrige, desinfizierende Gelzubereitung nach einem der vorangehenden Ansprüche 1 bis 9, die darüber hinaus mindestens einen Lösungsmittler umfasst.

11. Wässrige, desinfizierende Gelzubereitung nach Anspruch 10, worin der mindestens eine Lösungsvermittler in einer Menge im Bereich von 0,05 bis 2 Gew.-% enthalten ist.

12. Wässrige, desinfizierende Gelzubereitung nach Anspruch 10 oder Anspruch 11, worin der mindestens eine Lösungsvermittler ausgewählt ist aus der Gruppe, die besteht aus Glycerin, Propylenglykol, Sorbit, 1,3-Butylenglykol, Pentandiol, Polyethylenglykol, Hyaluronsäure, Harnstoff und Natriumpyrrolidoncarboxylat und Mischungen aus zwei oder mehreren davon.

13. Wässrige, desinfizierende Gelzubereitung nach einem der vorangehenden Ansprüche 1 bis 12, die darüber hinaus mindestens ein Ncutralisationsmittel umfasst.

14. Wässrige, desinfizierende Gelzubereitung nach Anspruch 13, worin die Menge des mindestens einen Neutralisationsmittels in einem Bereich von 0,1 bis 2 Gew.-% liegt.

15. Wässrige, desinfizierende Gelzubereitung nach Anspruch 13 oder Anspruch 14, worin die Gelzubereitung mit mindestens einem Neutralisationsmittel auf einen pH-Wert von 4 bis 9 neutralisiert ist.

16. Wässrige, desinfizierende Gelzubereitung nach einem der vorangehenden Ansprüche 14 bis 15, worin das mindestens eine Neutralisationsmittel ausgewählt ist aus der Gruppe, die besteht aus basischen organischen Verbindungen, vorzugsweise ausgewählt ist aus Isopropylamin, Aminomethylpropanol, Amino-methylpropandiol, Triethanolamin, Diisopropanolamin und/oder Tetrahydroxpropylethylendiamin und Mischungen aus zwei oder mehreren davon.

17. Wässrige, desinfizierende Gelzubereitung nach einem der vorangehenden Ansprüche 1 bis 16, worin die mindestens eine weitere desinfizierende Komponente ausgewählt ist aus der Gruppe, die besteht aus Invertseifen, Biguanidverbindungen, Phenolverbindungen, Iodverbindungen, Pigmentvcrbindungen und Mischungen aus zwei oder mehreren davon.

18. Wässrige, desinfizierende Gelzubereitung nach einem der vorangehenden Ansprüche 1 bis 17, die darüber hinaus weitere Komponenten umfasst, die gewählt sind aus der Gruppe, die besteht aus Rückfettern, Duftstoffen, etherischen Ölen, Farbstoffen, Konservierungsmitteln, und Filmbildnern.

19. Wässrige, desinfizierende Gelzubereitung nach einem der vorangehenden Ansprüche 1 bis 18, worin der Rückfetter ausgewählt ist aus der Gruppe, die besteht aus Glycerin, Fettsäurepolydiethanolamiden, Betain, Lanolin, Paraffinöl, Kokosfettsäurediethanolamid, Kokosfettsäuremonoethanolamid und Kombinationen davon.

20. Wässrigc, desinfizierende Gelzubereitung nach einem der vorangehenden Ansprüche 1 bis 19, worin die Menge an Wasser im Bereich von 15 bis 45 Gew.-% liegt.

21. Verfahren zur Herstellung einer wässrigen, desinfizierenden Gelzubereitung, das die folgenden Schritte umfasst, dass man
- die Alkoholkomponente mit der erforderlichen Menge Wasser mischt;
- in die Mischung die in alkoholisch wässriger Medien gut löslichen Wirkstoffkomponente zugibt;
- gegebenenfalls der Mischung einen Lösungsvermittler zusetzt und danach die weiteren Komponenten zugibt;
- nach Zugabe des Verdickungsmittels die Mischung homogenisiert und
- gegebenenfalls den pH-Wert einstellt.

22. Verwendung der wässrigen, desinfizierenden Gelzubereitung nach einem der Ansprüche 1 bis 20 zur Desinfektion der Haut und/oder der Hände.

23. Verfahren zur Desinfektion von Haut oder Händen, bei dem eine wässrige, desinfizierende Gelzubereitung nach einem der Ansprüche 1 bis 20 auf die zu desinfizierende Stelle aufgebracht und dort für eine vorbestimmte Zeit belassen wird.
